# EUROPEAN PATENT SPECIFICATION

(11) **EP 3 972 484 B1**
(45) Date of publication and mention of the grant of the patent: **27.08.2025**
(21) Application number: 20729148.5
(22) Date of filing: 19.05.2020
(51) Int. Cl.: A61B 5/00, A61B 5/1473

(54) **A SENSOR**
SENSOR
DÉTECTEUR

(30) Priority: 23.05.2019 GB 201907292
(43) Date of publication of application: 30.03.2022
(73) Proprietor: IP2IPO Innovations Limited, London N1C 4AG (GB)
(72) Inventor: BARBOT, Antoine, London Exhibition Road South Kensington London SW7 2AZ (GB); YANG, Guang-Zhong, London Exhibition Road South Kensington London SW7 2AZ (GB); WALES, Dominic, London Exhibition Road South Kensington London SW7 2AZ (GB); KIM, Jang Ah, London Exhibition Road South Kensington London SW7 2AZ (GB); ANASTASOVA-IVANOVA, Salzitsa Yordanova, London Exhibition Road South Kensington London SW7 2AZ (GB); TEMELKURAN, Burak, London Exhibition Road South Kensington London SW7 2AZ (GB); ABDELAZIZ, Mohamed EMK, London Exhibition Road South Kensington London SW7 2AZ (GB)
(74) Representative: Potter Clarkson
(86) International application number: PCT/GB2020/051217
(87) International publication number: WO 2020/234579

(56) References cited:
- WO-A1-2016/032335
- JP-A- 2003 232 725
- US-A1- 2009 244 542
- US-A1- 2011 306 854
- US-A1- 2016 022 159
- WIKIPEDIA: "Drawing (manufacturing)", 3 April 2019 (2019-04-03), XP055720980, Retrieved from the Internet <URL:https://en.wikipedia.org/wiki/Drawing_(manufacturing)> [retrieved on 20200807]

## Description

This invention relates to a sensor, and particularly, but not exclusively, to a sensor having combined electrical and optical sensors for use in *in-vivo* sensing.

A sensor of this type has particular application in the field of diagnostics. Sensors of this type are suitable for detecting diseases such as those identified below, although it is to be understood that sensors of this type could be used in other applications.

Such a sensor has application in the respiratory system of a human or animal. Diseases such as pneumonia, both typical and atypical, lung cancers, chronic pulmonary disease (COPD, including emphysema and chronic bronchitis), cystic fibrosis, asthma, tuberculosis, bronchiectasis, sarcoidosis and other diseases may be diagnosed using sensors of this type.

In the urinary tract, urethral cancers, bladder cancers, ureter cancers, kidney cancers, pyelonephritis, urinary tract infection and other diseases may be diagnosed.

WO 2016/032335 A1 discloses a sensor device comprising a needle with a needle tip comprising a sensor unit and a microfluidic channel structure with an orifice, wherein the orifice is especially arranged at the needle tip, for delivery or extraction of a fluid.

US 2016/0022159 A1 discloses an apparatus for directly measuring a blood pressure gradient, i.e. by real-time pressure measurements, using minimally-invasive techniques. The apparatus takes the form of a multi-sensory assembly, enclosed within a micro-catheter or a steerable guidewire, and comprises a plurality of optical pressure sensors arranged along a length of the distal end portion, for measuring pressure simultaneously at each sensor location.

US 2011/0306854 A1 discloses a syringe-based whispering gallery mode sensor includes a syringe including an assembly provided its needle, the assembly including an optical carrier having a reflective distal end, and at least one resonator coupled with the optical carrier.

US 2009/0244542 A1 discloses an integrated optical waveguide type surface plasmon resonance (SPR) sensor having an optical waveguide with a corresponding SPR sensing area, photodetectors, and wavelength tunable laser or any kind of external tunable laser source/coupler formed on a substrate. The integrated device may include a microfluidic structure for routing a target sample to the SPR sensor area. The microfluidic structure may include a mixer or a reaction chamber for mixing and allowing a physical or chemical reaction to occur, respectively.

According to the invention there is provided a sensor as defined in claim 1. Preferred embodiments of the invention are defined in the dependent claims.

By means of the present invention it is possible to carry out diagnostic tests *in-vivo.*

Because the sensor comprises a fibre, it may have very small dimensions, which facilitates insertion of the sensor into an appropriate part of the patient's body.

The sensor may have any convenient dimensions, and in some embodiments of the invention, the fibre forming the sensor is 0.3 m in length. In other embodiments of the invention, the fibre may be longer or shorter, and in some embodiments of the invention the fibre is approximately 1m in length.

The diameter of the fibre forming the sensor may be approximately 1mm, and in some embodiments of the invention it may be less. In some embodiments of the invention the diameter of the fibre is 0.2 mm.

In addition, because the sensing chamber is formed within a channel which is itself formed within the first fibre, the sensing chamber may be protected from the environment in which the sensor is positioned. In particular, the sensing chamber may be protected from damaging frictional contact with the surrounding environment.

In embodiments of the invention, the sensor further comprises a seal, sealingly connected to the fibre. The seal may comprise, for example a wall of the first fibre, or may comprise a seal formed from a polymer such as UV cured glue.

The seal assists in protecting the sensing chamber from the environment.

The first channel comprises a microfluidic flow channel or groove. The microfluidic flow channel enables microfluidic connections to take place along the length of the fibre. The first channel may have any convenient or desirable dimensions, and in some embodiments of the invention, the first channel has a diameter within the range 0.05 mm to 0.5 mm.

The first channel comprises features which may be either inherently formed within the channel, or may be formed separate thereto, which features result in microfluidic flow when a fluid passes through the channel.

In embodiments of the invention, the first channel comprises patterns etched into the wall of the first channel in the sensing chamber. These patterns will be referred to herein as microfluidic patterns. The microfluidic patterns cause microfluidic flow when a fluid is passed through the sensor.

The microfluidic patterns may have any desirable dimensions, and in some embodiments of the invention, the microfluidic patterns have dimensions of about 0.05 mm. In other embodiments of the invention microfluidic patterns may be smaller and larger, and in one embodiment of the invention microfluidic patterns have a dimension of 0.001 mm.

According to the invention, the sensor comprises a first pump operatively connected to the outlet of the sensor.

The first pump may be used in order to cause a fluid to flow through the sensor. A fluid may enter the sensor *via* the inlet and may then pass through the sensing chamber in order that fluid may be analysed by the sensing element which is operatively connected to the sensing chamber. By means of the first pump, the rate of flow of a liquid passing through the sensing element may be varied in order to suit the circumstances under which the sensor is being used.

In embodiments of the invention the first pump comprises a syringe pump.

In some embodiments of the invention the first pump comprises a reservoir pump operatively connected to a reservoir holding fluid.

In such embodiments of the invention, the pump is adapted to tune the pressure of the reservoir.

In some embodiments of the invention, there may be one or more syringe pumps and/or one or more reservoir pumps operatively connected to one or more reservoirs.

In embodiments of the invention comprising a syringe pump, the syringe pump may comprise a plunger operatively connected to a linear motor. The linear motor may be adapted to provide a constant speed to the plunger.

In such embodiments, there will be constant flow of fluid within the sensor. The flow may be either positive or negative depending on whether the plunger of the syringe is pushed or pulled by the linear motor. In other words, in such embodiments of the invention, the syringe pump may cause fluid to flow either into or out of the sensor.

In embodiments of the invention comprising a reservoir pump, the reservoir pump may be adapted to control pressure within a tuneable reservoir. If a positive pressure is applied by the reservoir pump, fluid contained in the reservoir will flow out of the reservoir. On the other hand, if a negative pressure is applied to the reservoir, fluid will flow into the reservoir.

In embodiments of the invention, the reservoir and the reservoir pump may be connected by means of a fluidic tube. There may be one or more such fluidic tubes. The fluidic tube(s) may be formed from polymer or glass, but other materials could also be used.

In embodiments of the invention, the one or more fluid tubes may be operatively connected to one or more channels extending between the inlet and the outlet of the sensor.

Fluid passing through the first channel and over the sensing element may be sensed by the sensing optical fibre.

The at least part of the or each sensing optical fibre is exposed within the sensing chamber. This means that fluid passing through the first channel will also pass over at least a part of the or each sensing optical fibre.

In embodiments of the invention, the sensor comprises a plurality of sensing optical fibres, each of which sensing optical fibres extends along the sensor such that at least a part of each sensing optical fibre is operatively connected to the first channel by means of the sensing chamber.

In such embodiments of the invention, at least one part of each of the sensing optical fibres may be exposed within the sensing chamber.

By having a plurality of sensing optical fibres, it is possible to optically sense and analyse a plurality of different variables at the same time in order to obtain a complete analysis relative to the diagnosis in question.

In embodiments of the invention the sensing element comprises an electrical sensor extending along the first fibre such that at least a part of the electrical sensor is operatively connected to the first channel by means of the sensing chamber.

The electrical sensor maybe in the form of an electrical conductor.

In embodiments of the invention where the electrical sensor comprises an electrical conductor, the electrical conductor may be in the form of wire. The wire may be contained within a sensing optical fibre or may be separate to any sensing optical fibres.

In embodiments of the invention the sensing element comprises a sensing optical fibre and an electrical sensor. In some embodiments of the invention, the sensing element comprises a plurality of sensing optical fibres and/or a plurality of electrical sensors.

According to the invention, the sensor further comprises a second channel extending along the sensor, which second channel is operatively connected to the first channel.

The second channel may be used as a cleaning channel and may therefore be used as a means for enabling a cleaning fluid to be passed through the sensor when the sensor is not in use. In other embodiments of the invention, the second channel may have a different purpose. In some embodiments of the invention, the first and second channels are both used to pass a fluid to be analysed through the sensor.

In embodiments of the invention comprising a first channel and a second channel, when the sensor is in a sensing mode, fluid from the ambient surroundings to be analysed may be pulled or drawn through both the first channel and the second channel after entering the sensor via the inlet.

In such embodiments of the invention, when the sensor is in a cleaning mode, a cleaning fluid may be flushed through the first channel via the outlet of the sensor and may pass through both the first channel and the second channel before emerging also through the outlet of the sensor.

The sensor may comprise a connector which connects the first channel to the second channel. The connector may be in the form of a connecting channel.

In embodiments of the invention the sensor may further comprise a second pump operatively connected to the second channel. In such embodiments of the invention, the first pump may be operatively connected to the first channel.

In embodiments of the invention comprising a second pump, the second pump may comprise a syringe pump or a reservoir pump of the type described here and above with reference to the first pump.

In such embodiments of the invention, the first and second pumps work together to create an appropriate flow of fluid through the sensor depending on, for example whether the sensor is in an operative mode or a cleaning mode.

In other embodiments of the invention, the first pump is operatively connected to both the first and second channels and serves to pump fluid through both the first and second channels as required. In such embodiments a single pump only is required.

In embodiments of the invention, the sensor comprises a third channel operatively connected to the first channel by means of the sensing chamber.

The third channel may be used in any convenient way, and in embodiments of the invention, the third channel is used to enable a reagent to be mixed with the fluid that is to be analysed by the sensor.

In embodiments of the invention, the sensor element comprises a first probe element removably positionable within the first channel.

In such embodiments of the invention, the sensing element is formed separately from and is removable from the first fibre.

This can be useful if, for example it is required to use different types of sensing elements. A first probe element may then be readily removed and replaced with a different first probe element in order to sense a different variable.

The first probe element may be formed from any suitable material and may for example be a drawn fibre.

In such embodiments of the invention, the first probe element is shaped such that when positioned within the first channel, voids are formed between the first channel and the probe element. In such embodiments of the invention, the microfluidic pattern forming part of the microfluidic flow channel is formed from the voids formed between the first probe element and the first channel.

In embodiments of the invention, the sensor comprises a second probe element adapted to be removably positionable within the second channel.

In embodiments of the invention the sensor comprises a light sensitive material operatively connected to one or more of the first, second and third channels. The light sensitive material may be light actuated, or may be any other material that expands when exposed to light.

In embodiments of the invention, the light sensitive material is patterned with a microfluidic pattern.

In some embodiments of the invention the sensor comprises a switch adapted to switch the sensor between a sensing configuration and a cleaning configuration. In embodiments of the invention in which the microfluidic flow channel comprises a light sensitive material, the switch may comprise a first switching optical fibre and a second switching optical fibre, the first switching optical fibre being operatively connectable to a cleaning channel and the sensing element, and the second switching optical fibre being operatively connected to a drain channel and the sensing element.

In such embodiments of the invention, the sensor may comprise an end portion at an end of the sensor, which end portion is formed from the light sensitive material.

The light sensitive material may be a light actuated material, such as a thermal actuated polymer of the type described in International patent application No. WO 2012/142235 and WO 2017/120594. Alternatively the light sensitive material may be any other material that is adapted to expand when exposed to light.

The light sensitive material is adapted to fit over an exposed end of the sensor and thus has dimensions that are similar to the cross-sectional dimensions of the sensor.

The end portion may be patterned with a microfluidic circuit. The microfluidic circuit may be made by moulding polymer during its curing or ablation with lasers, FIB or classical milling.

In use, in order to switch the sensor into the sensing configuration, light may be shone on the first switching optical fibre. This causes the light sensitive material to expand, thereby blocking a flow path from a sensing element to the cleaning channel. When it is required to switch the sensor in the cleaning configuration, light is shone on the second switching optical fibre. This causes the light sensitive material to expand over the second switching optical fibre, thus blocking the flow path between a sensing element and the drain channel.

In embodiments of the invention the sensor further comprises a data analysis unit connectable to a proximal end of the first fibre, wherein the sensing optical fibre operatively connects the sensing element to the data analysis unit.

The invention will now be further described by way of example only with reference to the accompanying drawings in which:
Figure 1 is a schematic representation of a sensor according to a first embodiment of the invention;
Figure 2 is a cross sectional representation of the sensor of Figure 1;
Figure 3 is a schematic representation of a diagnostic system incorporating a sensor according to embodiments of the invention;
Figure 4 is a schematic representation of the proximal end of a sensor according to embodiments of the invention;
Figure 5 is a schematic representation of the proximal end of another embodiment of a sensor according to the invention;
Figure 6 is a schematic representation a sensor according to another embodiment of the invention;
Figure 7 is a cross sectional representation of the sensor of Figure 6;
Figures 8, 9 and 10 are cross sectional representations taken along A-A, B-B and C-C respectively as shown in Figure 7;
Figure 11 is a schematic representation of part of the sensor of Figure 6 showing the sensor in a sensing configuration;
Figure 12 is a schematic representation of part of the sensor in Figure 6 shown in a cleaning configuration;
Figures 13 to 15 are schematic representations of a sensor according to another embodiment of the invention having three channels;
Figures 16 and 17 are schematic representations of the sensor shown in Figures 13 to 15 in a sensing configuration and a cleaning configuration respectfully;
Figure 18 is a schematic representation of a portion of a sensor of the type shown Figures 13 to 15 and comprising a microfluidic mixture;
Figure 19 is a schematic representation of a sensor according to another embodiment of the invention comprising two channels;
Figure 20 is an exploded perspective view of the sensor of Figure 19;
Figure 21 is a top view of the sensor of Figure 19;
Figure 22 is a perspective view of the tip of the sensor of Figure 20 showing fluid inlets forming part of the sensor;
Figure 23 is a schematic representation of the sensor of Figure 19 showing the sensing regions;
Figure 24 is a detailed perspective view of the tip of a sensor according to another embodiment of the invention and having an optically controlled valve;
Figure 25 is a schematic representation of the valve forming part of the sensor tip shown in Figure 24 in an open position;
Figure 26 is a schematic representation of the valve of Figure 25 in a closed position;
Figures 27 and 28 are schematic views from above of the sensor of Figure 28 in the sensing configuration cleaning configuration respectively; and
Figure 29 is schematic representation showing flow of fluid through the embodiment of the invention shown in Figure 11.

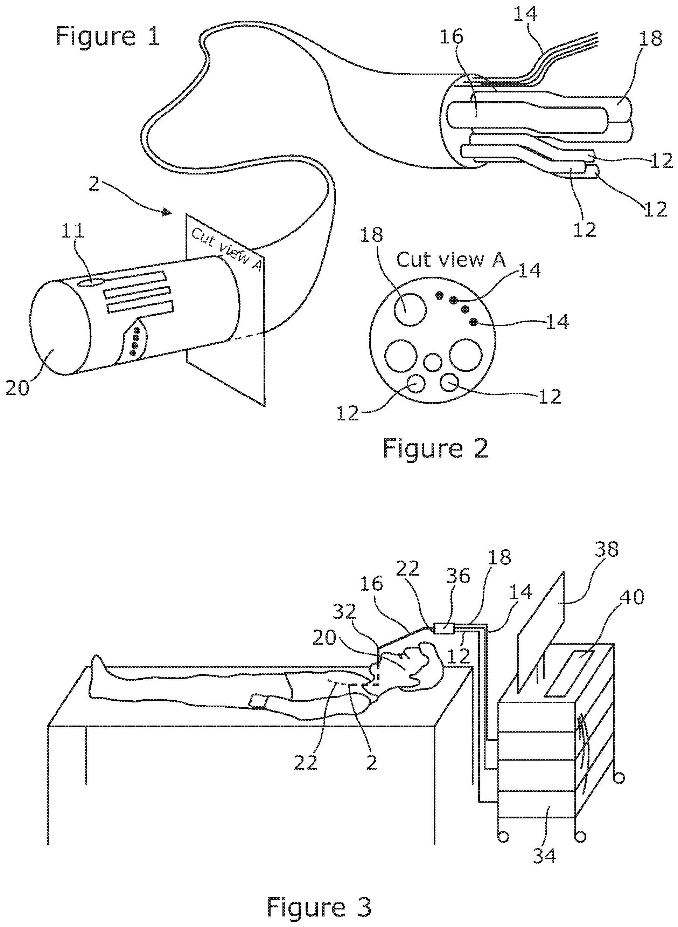

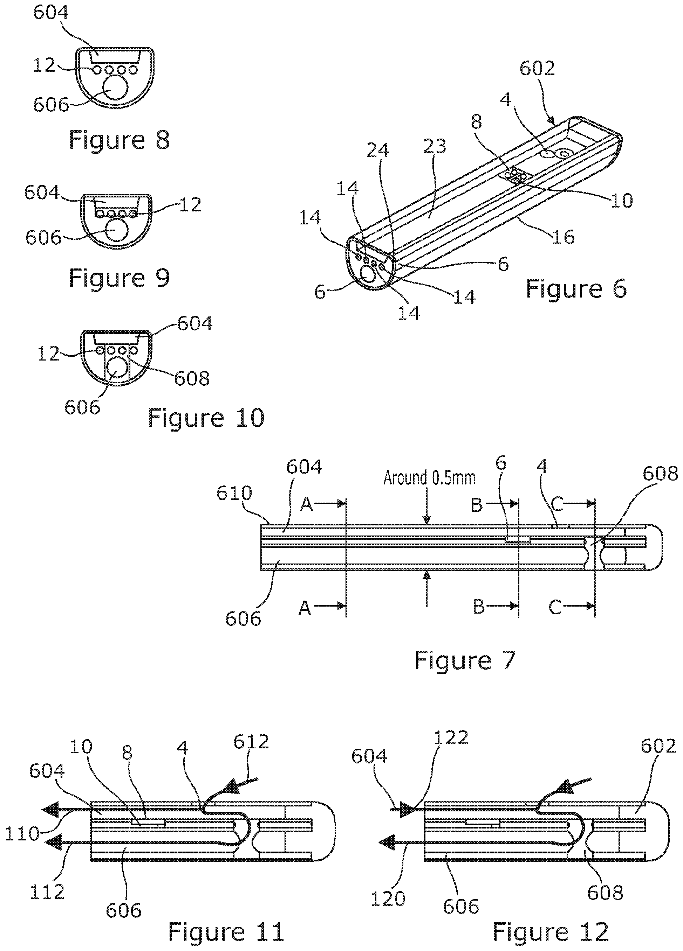

Referring first to Figures 1 and 6 to 12 a sensor according to an embodiment of the invention is designated generally by the reference numeral 2. The sensor comprises a microfluidic sensor comprising a distal end 20 and a proximal end 22 and an inlet 4 and an outlet 6 as shown particularly in Figure 7. The sensor 2 also comprises a sensing chamber 8 positioned between the inlet 4 and the outlet 6, and a sensing element 10. In this embodiment of the invention, the sensing element comprises a plurality of sensing optical fibres 12 and electrical wires 14, each of which is partially exposed within the sensing chamber 8.

The sensor 2 comprises a fibre 16 formed from a drawable material. In order to form the sensor 2, the fibre 16 is drawn in the shape shown specifically in Figure 6 from a preform.

The optical sensors 12 and/or electrical wires 14 are placed inside the fibre, either by co-feeding during the drawing process, or by sliding in after the drawing process.

Once the electrical wires and/or optical sensors have been placed inside the sensor 2, the wires and/or optical sensors may be exposed by removing some of the fibre material to expose the sensing chamber.

The microfluidic patterning may be achieve using any convenient method such as laser patterning, FIB patterning, moulding, micro-milling. Other methods may also be appropriate.

The sensor 2 is adapted to sense variables either electrically or optically by means of the sensing optical fibres 12 and the electrical wires 14. Sensing is achieved by the functionalisation of a surface of the sensor 2.

The sensing elements may be positioned anywhere along the length of the sensor 2. For example, the sensing chamber may be positioned anywhere along the length of the sensor 2 and/or at a distal end 20 of the sensor 2. By means of the present invention therefore it has been possible to design a microfluidic chip that is connected to both a measurement unit and fluid input all on a single fibre 16. The input of the sensor 2 allows liquid sampling to take place *in vivo.* The sensing is incorporated inside the sensing chamber 8 by a functionalisation of the surface of the sensor 2 which is in the form of a microfluidic chip.

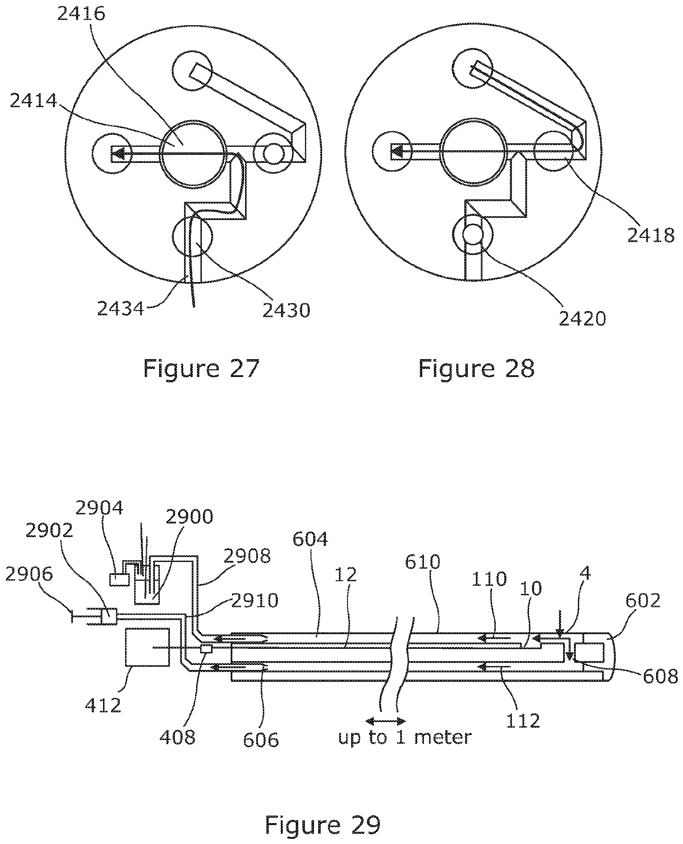

The sensor 2 is connected at the proximal end 22 to a tunable pressure reservoir 2900 (shown in Figure 29) in order to generate a constant positive or negative pressure.

By means of the sensing optical fibres 12 and the electrodes 14, measurements may be made electrically and/or optically.

By means of the present invention therefore the sensor 2 may be used for *in vivo* chemical sensing and a distal end of the fibre 16 may be positioned at a point where measurements are to be taken.

Because all the components of the sensor 2 are within a single fibre 16, a controlled laboratory-like environment is achieved for the sensing process. This helps to reduce or eliminate noise and other interactions that may adversely affect open sensors. In addition, the sensing chamber 8 is protected from mechanical damage during insertion and throughout the measurement process.

By means of the present invention, all connections are provided through a single fibre and thus the sensor 2 is compact and robust.

The sensing environment passing through the sensing chamber is constant and known thus allowing repeatable and quantifiable measurements.

The invention may comprise a multi-material fibre 16 having inherent electrical, optical and fluidic channels formed therein with desired geometries.

The sensor 2 may be made using any convenient methods such as focussed ion beam (FIB), laser patterning, drilling and milling. Such processes may be used to add non-axial features to the fibre allowing channel connection or complex microfluidic features to be incorporated into the sensor 2. The sensor 2 further comprises fluid tubes 18 for allowing fluid to pass through the sensor 2.

A sensor 2 according to embodiments of the invention has a wide range of applications but is of particular use within the medical field.

Referring to Figure 3, a medical diagnostic system 30 comprising a sensor 2 of the type shown in Figure 1 is illustrated schematically. In the illustrated embodiment, a catheter 32 serves to connect the sensor 2 to an analyser 34. The catheter 32 is connected to an interface box 36, and the sensor 2 extends through the catheter to the fibre interface box 36. The optical fibres 12, electric wires 14 and fluidic tubes 18 pass through the fibre interface box 36 to the analyser 34. The analyser 34 comprises a user interface 38, 40 which may be in the form of a monitor 38 and keyboard 40 for example.

The size of components such as the user interface 38, 40 and the analyser 34 may vary to suit the application to which the sensor 2 is being put.

In the illustrated embodiment, the sensor 2 has been inserted into the lungs of a patient via the mouth of a patient. However, a sensor according to embodiments of the invention may be adapted to be inserted through other orifices, natural or otherwise, in order to take measurements in an appropriate part of the body.

As may be seen particularly from Figure 3, the proximal end 22 of the fibre 16 is connected to the fibre interface box 36, and the distal end 20 of the fibre 16 is positioned appropriately within the body of the patient.

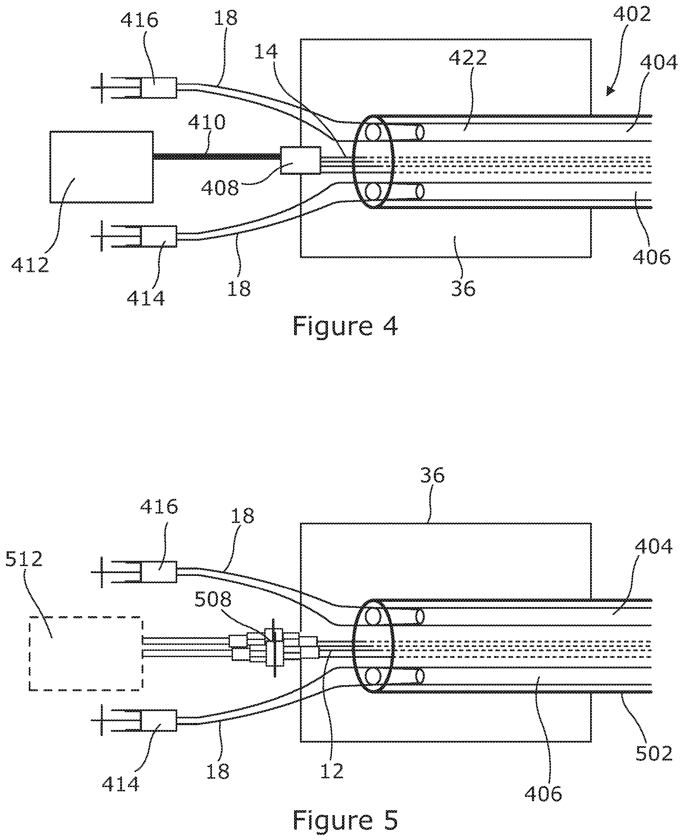

Referring now to Figures 4 and 5 the proximal end of a sensor accords to two embodiments of the invention is shown in more detail in order to illustrate how a sensor 2 according to embodiments of the invention may be connected to the fibre interface box 36.

Referring first to Figure 4, the proximal end 422 of a sensor 402 according to embodiments of the invention is illustrated. In this embodiment of the invention, the sensor 402 comprises a first channel 404 and a second channel 406. The channels 404, 406 will be described in more detail below.

The fibre further comprises electrical wires 14 which are connected via an electrical connector 408 and a connection cable 410 to an electrical measurement station 412.

Fluidic tubes 18 are connected to syringe pumps 414 and 416 respectively.

Turning now to Figure 5, a sensor 502 according to another embodiment of the invention is illustrated. Parts of the sensor 502 which are similar to those of the sensor 402 have been given corresponding reference numerals for ease of reference.

In this embodiment of the invention the sensor 502 comprises optical fibres 12 which are connected via optical fibres connectors 508 to an optical spectroscopy set up 512.

In some embodiments of the invention both electrical wires (or electrodes) 14 and optical fibres 12 will be present in a sensor according to embodiments of the invention. Such a sensor may comprise a multi-material fibre having inherent electrical, optical and fluidic channels formed therein with desired geometries.

Referring now to Figures 6 to 12 and 29 an embodiment of the invention in the form a sensor 602 is illustrated. In this embodiment of the invention the sensor 602 comprises inlet 4 and two outlets 6. Positioned between the inlet 4 and the outlets 6 is a sensing chamber 8 with a sensing element 10 exposed therein. The sensing element 10 comprises portions of electrical wires 14.

The sensor 602 also comprises a first channel 604 which forms a microfluidic channel and a second channel 606. The first and second channels 604, 606 are connected by a connecting channel 608. The sensor 602 is sealed by a cover 23 formed from heat shrink polymer forming a seal 610. This allows easy machining of the surface of the sensor 602 using techniques such as focus ion beam or laser patterning.

The first channel 604 is operatively connected to the inlet 4 and to first outlet 6. The second channel 606 is operatively connected to the first channel 604 by means of the connecting channel 608 and is also connected to the second outlet 6. One or more pumps of the type shown in Figures 4 and 5 are operatively connected to each of the channels 604, 606.

In order the functionalise the electrodes 14, a surface of each of the electrodes is cleaned electrochemically with 50 mM sulphuric acid. The electrodes are then dried and a layer of conductive platinum nanoparticles is deposited on the electrodes to increase the surface area of the electrodes.

Next, an ion-sensitive cocktail containing ionic sites such as nitrophenyl octyl ether, ionophore specific for the specific analyte of interest such as pH ionophore, sodium, potassium, calcium ionophores, etc.

In the case of enzyme sensing, a different cocktail is drop casted with an enzyme that is sensitive towards the analyte of interest which is crosslinked to bovine serum albumin using glutaraldehyde. Several layers of biocompatible membrane layers such as polyurethane are deposited at the end to achieve protection and long life-time response.

In order to ensure a good seal, the cover 23 may be made from a heat shrinkable polymer. In such embodiments of the invention, the sensor 2 is first of all fit with a loose fitting heat shrinkable cover. The sensor is then heated so that the cover shrinks tightly around the fibre.

In other embodiments, the step of functionalisation may be carried out after the sensor 2 has been sealed.

In order to take a measurement, fluid from the surrounding environment is caused to enter the sensor 602 via inlet 4 and is drawn through the sensing chamber 8 over the sensing elements 10 by means of the one or more pumps (shown in more detail in Figure 29). In the sensing configuration, as shown in Figure 11, fluid to be sampled is caused to flow along both the first and second channels 604, 606 as shown by the arrows 110, 112 in Figure 11.

In this embodiment of the invention, the first channel 602 is connected at its proximal end to a tuneable pressure reservoir 2900 (Figure 29) in order to generate a constant positive or negative pressure.

In the sensing configuration, the electrodes are used to carry out electrochemical measurements in order to analyse the liquid being drawn through the sensor 602.

In the sensing configuration, as shown in Figure 11, the one or more pumps create suction through channel 604 and 606 in order to pull, or suck fluid to be analysed from the surrounding environment into the sensor 602 via inlet 4 and through the first and second channels 604, 606 as shown in Figure 11.

By setting a negative pressure, external liquid will flow inside the channel from the opening 24 and will pass over the electrodes 14.

In contrast, when the sensor 602 is in the cleaning configuration, as shown in Figure 12, the one or more pumps cause suction through channel 606 and flushing through channel 604 as shown by the arrows 120, 122. A cleaning solution may be pulled into the sensor via inlet 4 and channel 604, and may be drawn through the sensor and out through channel 606. Because of the flow created in this way, there will be no leakage of the cleaning solution into the environment due to the flushing taking place through channel 604 which prevents cleaning fluid from exiting via inlet 4.

In the cleaning mode, the reservoir may be filled with a cleaning solution which will flow through channel 604 and through the opening 24 into the *in vivo* environment. The cleaning fluid will therefore need to be a biocompatible solution such as a saline solution.

A cleaning solution may be pulled through the sensor by increasing the pressure of the reservoir to a positive pressure.

The flow inside the sensor 602 is shown in more detail in Figure 29.

The flow with the sensor 602 may be controlled either by syringe pumps 2902 or by reservoir pumps 2904 which tune the pressure of the reservoir 2900. In some embodiments of the invention there may be a mixture of syringe pumps 2902 and reservoir pumps 2904.

In this embodiment of the invention each of the channels 604, 606 is connected to either a syringe pump 2902 or a reservoir 2900.

The syringe pump 2902 comprises a plunger 2906, the movement of which is controlled by a linear motor (not shown). The linear motor provides a constant speed to the plunger which in turn causes a constant flow of fluid outside the syringe pump 2902. The flow may be either positive or negative depending on whether the plunger 2906 is pushed or pulled by the linear motor.

The reservoir pump 2904 controls the pressure of air inside the reservoir 2900 which is sealed. The pressure acts on fluid within the reservoir 2900 to cause a flow of fluid. If a positive pressure is applied by the pump 2904, fluid will flow out of the reservoir 2900 towards the sensor 602.

If a negative pressure is applied by the reservoir pump 2904, fluid will flow from the sensor 602 to the reservoir 2900.

The reservoir 2900 and the syringe pump 2902 are connected to the sensor 602 by means of fluid tubes 2908 and 2910 respectively. The tubes 2908, 2910 may be made from any suitable material such as polymer or glass although other materials may also be suitable. The tubes 2908, 2910 are connected to the channels 604, 606 respectively. A seal is formed between a respective fluid tube 2908, 2910, and channel to 604, 606 in order to ensure efficient flow of fluid between the reservoir 2900, syringe pump 2902 and the sensor 602.

In the embodiment shown in Figure 29, fluid enters the sensor 602 via inlet 4 and then flows via channels 604, 606 towards the reservoir 2904 and syringe pump 2902.

The sensor 602 may be used as an electrochemical sensing mechanism.

Electrochemical detection of different targets such as electrolytes and biomolecules can be realised onto one platform by using the sensor 602. The microfluidics forming part of the sensor 602 ensure a better control of the fluids at the surface of the electrodes.

The principle of work involves a setup where the potential or indicator electrode is measured against a reference electrode under zero-current conditions. Solid-state ion selective electrodes are based on low soluble salts of the ion of interest. Changes of the transmembrane potential is proportional to the analyte concentration.

In the case of biomolecule detection, the indicator electrode has an enzymatic layer and outer protective layer limiting our working range. Detection of the changes in the current output is proportional to the concentration of the analyte of interest.

Another important method of electrochemical detection is through the immobilisation of an antibody onto the electrode surface which has an effect on the amount of the immobilised protein and in current signal of the protein. Microfluidic -based electrochemical sensing is a very sensitive, rapid and specific way of detection. Changes in the current output with time is proportional to the analyte concentration. In one embodiment of the invention the sensor may be used as an affinity biosensor which comprises a biological recognition element such as an antibody, receptor protein, biomimetic material, or DNA interfaced to a signal transducer, where the measured signal is related to the concentration of an analyte. The electrochemical detection offers a less expensive means of reading the signal. If the electrochemical reporters and the electrolyte are chosen correctly, the electrical signal is stable over time and may have less interferences compared with optical detection.

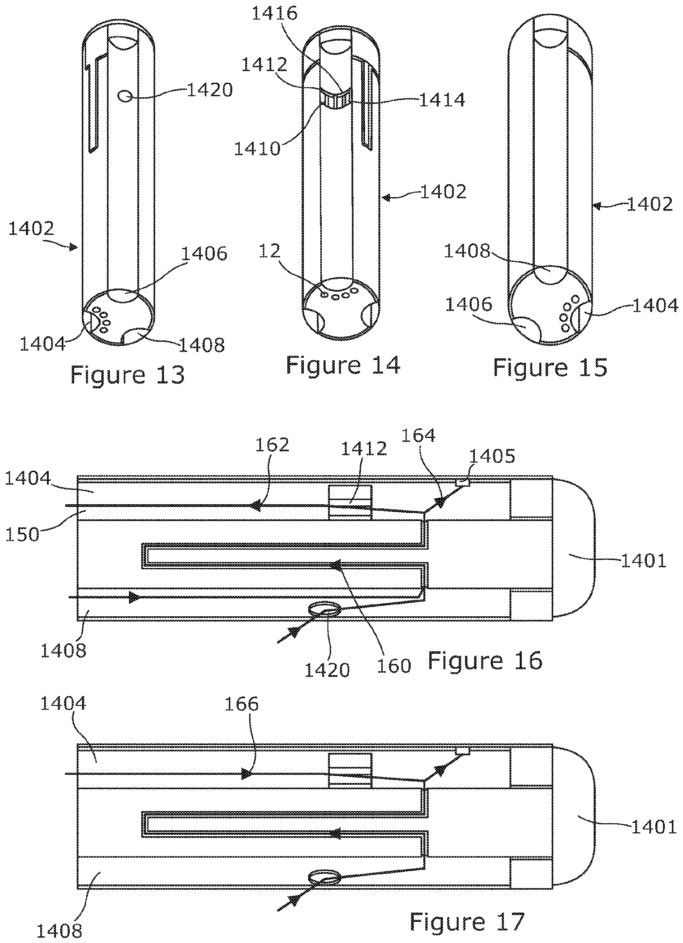

Referring now to Figures 13 to 18 another embodiment of the invention is illustrated. This embodiment comprises a sensor 1402 comprising three channels 1404, 1406 and 1408. The sensor 1402 is formed using a similar method to that described hereinabove with reference to the embodiment shown in Figures 6 to 12. Each of the channels 1404, 1406 and 1408 is sealed by means of a heat shrink polymer. The sensor 1402 further comprises a sensor tip portion 1401 which is sealed by any convenient means for example by applying a liquid polymer drop at the tip 1401. The polymer will naturally fit the microfluidic channels by capillary forces depending on choices of polymers, it may be cured, (solidified) with time, heat or UV exposure.

Once the heat shrink polymer and the polymer at the tip of the sensor have been applied, the heat shrink polymer is pierced to form an aperture 1420. This allows an input from the external environment to the microfluidic.

A first channel 1404 has formed therein a microfluidic flow structure 1410 and a sensing chamber 1412 containing a sensing element 1414 extending therethrough. The channel 1404 is similar to the channel 604 described hereinabove with reference to Figures 6 to 12. The sensor 1402 comprises a plurality of sensing optical fibres 1412 portions of which are exposed within the sensing chamber 1412 to act as a sensing element 1414. The sensing chamber 1412 may also comprise electrodes 1416 in order that electrical conductors may also be used to form part of the sensing element 1414.

Each of the channels 1404, 1406, 1408 are operably connected to one another via connector 1405. Channel 1408 serves as a drain channel and channel 1406 serves as a reagent channel.

The channels 1404 and 1406 are connected to a tuneable pressure reservoir (Figure 29) in order to generate a constant positive or negative pressure. The connector 1405 is connected to channel 1408, and this channel is also connected to the pressurised reservoir.

The pressurised reservoir connects to channel 1408 such that the channel 1408 is filled with a liquid which mixes with liquid surrounding the sensor 1402. The mixing liquid may be, for example an anticoagulant to prevent blocking of the microfluidic sensor in situations where the sensing environment comprises blood and/or protein which fixes to a particular bioelement and which may be detected by means of electrode 1412.

A negative pressure of the reservoir is set on the input connected to channel 1404 in order to direct the mixed flow to the sensing region 1408 of the sensor 1402.

Electrochemical sensing may then take place by means of the electrodes 1412.

In the sensing configuration as shown in Figure 16, fluid to be analysed is drawn into the sensor via inlet 1402. The fluid sample drawn in in this way will pass through the sensor on a path indicated by arrows 160, through the mixing area where additives may be mixed with the sample and then along through channel 1404 and through the sensing area 1412 before exiting via an outlet 150 at an end of the channel 1404.

The pathway 160, 162 is relatively long. This ensures that the fluid sample with which the channel is filled is thoroughly mixed. The sensor 1402 ensures a non-turbulent flow of fluid within it, and therefore a long path is required to enable the mixing to take place via diffusion processes.

A more convoluted shape for the pathway 160 could be used in other embodiments in order to improve diffusive mixing processes.

Some of the sample of fluid may also exit via the drainage channel 1406 as indicated by arrow 164.

In the sensing configuration, a reagent or other additive may enter the sensor in 1402 via an inlet at an end of the channel 1408. The reagent may then be pulled through the sensor by means of a pump to mix with the sample to be analysed to pass through the sensor along the same path identified by arrows 160, 162 as described hereinabove with reference to the fluid sample.

Channel 1408 may thus be used to mix for example an additive with the sample before the sample is tested.

In a cleaning configuration as shown in Figure 17, the sensing channel 1404 may also be used to allow a cleaning solution to pass through the sensor 1402 when the sensor is not being used to measure a sample. In the cleaning configuration cleaning solution will be drawn through the sensor 1402 by means of a pump (not shown) to a pass through the sensor 1402 in the direction of arrow 166.

This may be achieved by setting a negative pressure by means of the tuneable pressure reservoir.

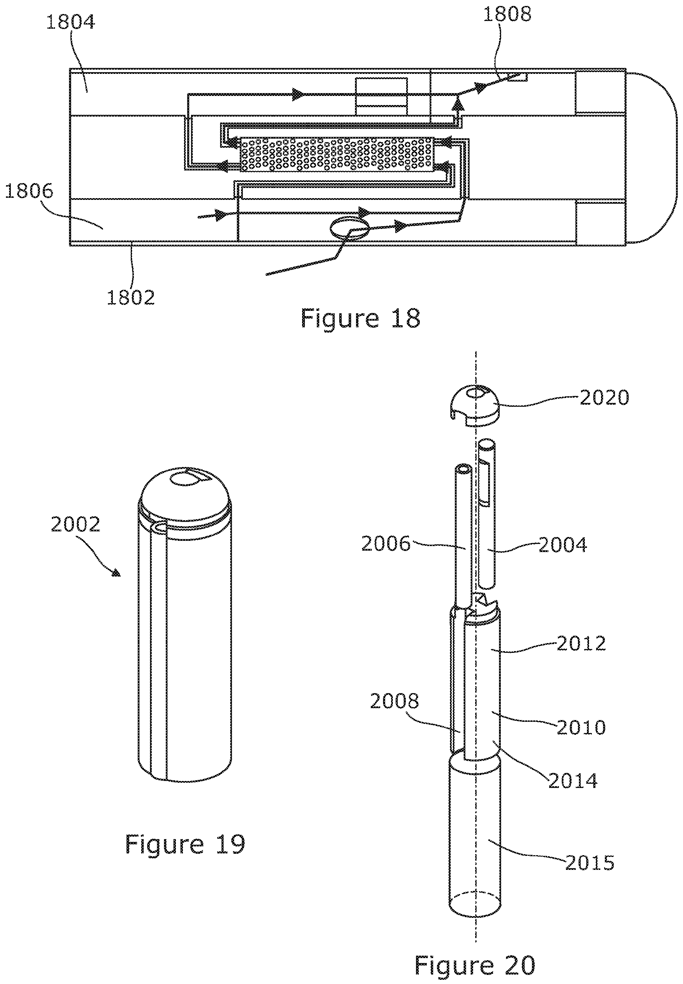

Turning now to Figure 18, an embodiment of the invention which is suitable for the separation of red blood cells and platelets from white cells and circulating tumour cells is shown. The sensor in this embodiment is designated generally by the reference numeral 1802. This embodiment is similar to the embodiment shown in Figures 13 to 17 in that the sensor 1802 comprises three channels 1804, 1806 and 1808. Channel 1804 is a cleaning channel, channel 1806 is a buffer channel.

The sensor 1802 is formed using the method described hereinabove with reference to previous embodiments. Specifically, the sensor 1802 is formed by drawing a fibre containing four electrodes or optical fibres such that these electrodes or optical fibres are positioned beneath one of the channels 1804, 1806, 1808.

Focussed ion beam (FIB) is used to open the fibre to form a window in order to position an electrode at an appropriate position within one of the channels.

The end of the channel may then be sealed with UV curable resist.

The electrodes and/or optical fibres can then be functionalised.

In this embodiment of the invention, the channels are isolated from the external environment through use of a heat shrink polymer.

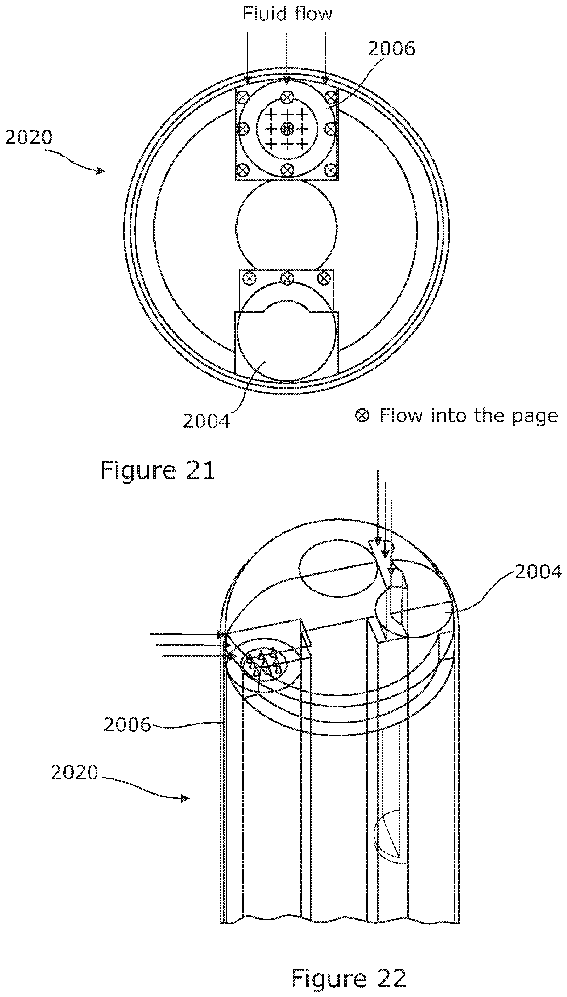

Referring now to Figures 19 to 23, a sensor 2002 according to another embodiment of the invention is illustrated schematically.

The sensor 2002 is adapted to work with a constant flow of fluid that is to be analysed and is able to continuously sense the fluid passing through the sensor 2002.

The sensor 2002 comprises a side sensing optical fibre 2004, and a tip sensing optical fibre 2006. The tip sensing optical fibre 2006 is formed from a multimode optical fibre cut to an appropriate length. The length may be between 10 and 15cm for use in shallow regions such as the oral cavity, nasal cavity, brain, open incision etc., and between 30cm to 1 m for use in deep area such as the lungs, intestines, liver, stomach etc.

The polymer protective jacket surrounding the fibre may then be removed using a fibre stripper. Next the end tips of the optical fibre may be cleave using a fibre cleaver.

The fibre is then placed and clamped within a 3D printed fibre holder. Direct laser writing, or two photon polymerisation (2PP) of photo resist of microstructures on the tip of the fibre with femtosecond near infrared laser takes place. After that step, the development of the polymerised microstructures by emersion in a developer such as propylene glycol methyl ether acetate takes place.

Next, the microstructure is metallised with a thin layer of approximately 100nm of a noble metal such as gold or silver by metal deposition techniques. Suitable techniques include electron beam deposition, thermal evaporation, sputtering etc.

After these steps have been carried out, the fibre is ready to act as the sensor 2006 shown in Figure 20.

The side sensing optical fibre 2004 is formed using similar steps, up to the point where the fibre may be placed and clamped within a 3D printer fibre holder.

At this point, a length of the cladding of the optical fibre having a length approximately 1 to 2cm is removed to expose the core of the fibre. The cladding may be removed all around the core, or just in one direction. The cladding may be removed using any suitable technique such as chemical etching using ammonium fluoride or hydrofluoric acid, etc, or by mechanical polishing or milling techniques.

A section of the exposed core is then coated with a layer of a metal or with multiple layers of different metals including, but not limited to: gold; silver; platinum and copper.

If gold is used, an initial layer of chromium of approximately 5nm thickness may be deposited to the core of the fibre before the gold is deposited in order to facilitate proper adhesion of the gold layer to the fibre.

By following these steps the sensor 2004 is formed.

In this embodiment of the invention, the tip sensing optical fibre 2006 is used to carry out Surface Enhanced Raman Spectroscopy sensing (SERS) whilst the side sensing optical fibre 2004 is adapted to perform Surface Plasmon Resonance (SPR) sensing.

SPR optical sensing is based on the change of local refractive index. This means that fibres adapted to sense this way must be chemically functionalised in order to detect an particular element.

SERS sensing on the other hand measures a characteristic spectrum that can be matched to a database to identify a particular element. This means that SERS can be used without any functionalisation. However, functionalisation can still be performed to increase the sensitivity of the measurement to a particular element such as bacteria, a cell or protein.

In order to functionalise the fibres the topmost metal layer of the sensing region is coated with chemical, biochemical or nanoparticle moeities that are intended to sense the analyte of interest by exposing the metal coated region to a solution/suspension of these materials. The moieties for chemical sensing could include, but are not limited to; crown ethers, calixarenes, other synthetic ionophores, dyes, etc. Biochemical moieties for biological sensing could include, but are not limited to, antibodies, antigens, proteins, biological ionophores, etc. The nanoparticles would facilitate LSPR (localised surface plasmon resonance) sensing and these nanoparticles could be gold, silver, etc. Furthermore, these nanoparticles would then be functionalised with chemical or biochemical moieties for sensing. Solutions/suspensions of chemicals and/or biochemicals to enable sensing will be used to functionalise the metal coated area/s of the optical fibres using standard chemical techniques. Then cleaning/washing steps with solvents or water or biological buffer solutions will be achieved using standard chemical techniques, to ensure the functionalised sensing region/nanoparticle region is prepared for analyte sensing.

This functionalisation step can be made before the assembly in the main fibre but also after step 6 below by using the microfluidic connection of the fibre to provide the coating solution as well as the rinsing solution.

The sensor 2002 may be assembled using the steps set out below:
1. Drawing a micro-channel + supporting fibre (2010)
2. Sitting the sensor fibres in the channels (2008). The channels are designed larger than the fibre so a spacing remain allowing a microfluidic canal between the channel and the fibre
3. Insertion of the assembly of 2004, 2006, and 2010 in the heat shrink tubing 2015
4. Closing the head of the assembly with a micro-machined or 3D-printed cap 2020, which has desired openings for fluid delivery
5. Heat shrink the heat shrink tubing 2015
6. This assembly becomes the component 2002 in Figure 19

Together the fibres 2004 and 2006 form part of the sensing element of sensor 2002. The optical fibres 2004 and 2006 are shaped to fit within channels 2008 formed in body portion 2010 of the sensor 2002.

The shape of the fibres 2004 and 2006 relative to the shape of the channels 2008 is such that when the fibres 2004 and 2006 are positioned within the channels 2008, microfluidic channel is formed within each of the channels 2008 by the gaps existing between the fibres 2004, 2006 and a respective channel 2008. The channels 2008 are formed within a fibre 2012 in which microfluidic channel grooves are formed. These microfluidic channel grooves form the sensing chamber in the sensor 2002.

In the illustrated embodiment, when the channel 2008 is designed such that it becomes deeper towards a bottom end 2014 of the body portion 2010. This results in the fibre 2006 being held away from the groove wall forming the microfluidic channel.

For the tip sensing optical fibre 2006, a sensing chamber is formed as will be described hereinbelow.

In this embodiment of the invention, the sensor further comprises a cap 2020 engageable with each of the fibres 2004, 2006. Fluid inlets for both fibres 2004, 2006 are formed on the cap 2020. Axial and side openings are designed on the fibres in order to ensure that the flow of fluid from outside of the sensor passes over the sensing region. The diameter of the axial and side openings may be adjusted in order to ensure an appropriate intake flow rate. The smaller opening will result in a smaller flow rate.

A constant flow of fluid is achieved through use of syringe or pressure pump at a proximal end of the sensor. The constant flow is achieved by creating a negative pressure through the sensor 2002. This results in a constant flow of fluid flowing from the surrounding environment through inlets passing into the tip sensing region and a side sensing region respectively.

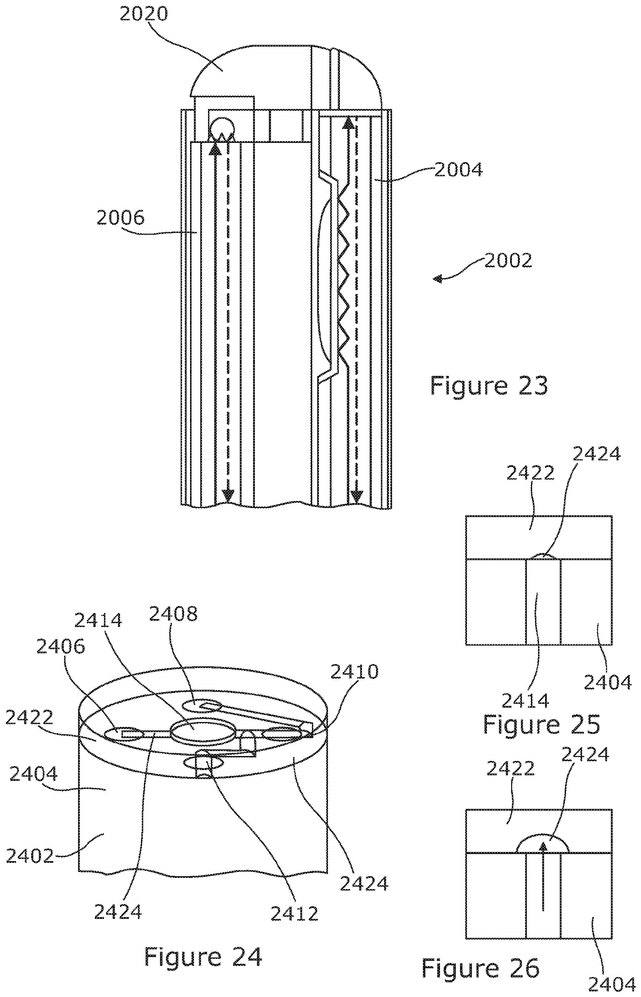

Referring now to Figures 24 to 28, a sensor 2402 according to another embodiment of the invention is illustrated.

The sensor 2402 is formed from a polymer fibre 2404 and is formed with channels (in this case 5 channels) 2406, 2408, 2410, 2412 and 2414. A fibre 2416 is placed within channel 2414. In this embodiment of the invention, the fibre is prepared to sense with the tip and is therefore similar to the probe 2006 shown in Figures 19 to 23 and described above. Fibres 2418 and 2420 are fitted into channels 2410 and 2412 respectively.

In order to maintain the fibres in place in the respective channels, as well as to prevent any leakage, glue is used to fill any spaces between a respective fibre and the channel in which it is held. An end portion 2422 is formed from a light actuated material such as a thermal actuated polymer or any other material that expands when it is exposed to light. The end portion 2422 is cut to fit over an end face 2424 of the sensor 2402.

The end portion 2422 is patterned with a microfluidic pattern which is similar to the microfluidic patterns described hereinabove with respect to the previous embodiments. This results in a microfluidic channel 2424 being formed.

In this embodiment, the microfluidic pattern is formed by moulding the polymer forming the end portion 2422 during curing or by ablation with lasers, FIB or classical milling.

The end portion 2422 may be fitted to the sensor 2402 by any convenient means such as by using an adhesive.

The channel 2406 is connected to a depression pump (not shown) to constantly generate a vacuum force in order to attract liquid to be tested into the sensor 2402. The channel 2408 is connected to a cleaning liquid at ambient pressure.

Referring now to Figure 25 no light is shone through fibre 2420which is fitted into channel 2412. This means that the polymer end portion 2422 maintains the shape shown in Figure 25. This in turn means that liquid may pass through channel 2414.

Turning now to Figure 26, light is shone through optical fibre 2420 in channel 2412. This results in the expansion of the polymer forming the end portion 2422 in a region close to the tip 2424 of the fibre 2416. This expansion results in the polymer forming the end portion 2422 filling the channel space and thereby blocking the channel 2414. In this situation no fluid can pass through the channel 2414.

During sensing, light directed via the fibre 2420 is switched off. This creates a channel between the outside of the microfluidic chip 2430 and end portion 2422. This allows liquid surrounding the sensor 2402 to enter the sensor via an opening 2434 and to then pass through the sensing region created in the end portion 2422 of the sensor 2402 as shown in Figure 27.

During the cleaning step, light is initially directed onto fibre 2420 to close the connection between the sensing region and the ambient surroundings.

At this point light directed onto fibre 2418 is stopped in order to open the access between channel 2406 which acts as a drain channel, and channel 2408. This causes cleaning liquid to be pulled into channel 2406 then to pass over the sensing region as shown in Figure 28.

When the sensing process is completed, light is then directed onto fibre 2418 in order to stop the flow of the cleaning liquid.

## Claims

1. A sensor (2) comprising an inlet (4) and an outlet (6), a sensing chamber (8) positioned between the inlet (4) and the outlet (6), and a sensing element (10) operatively connected to the sensing chamber (8), wherein:
the sensor (2) comprises a first fibre (16) formed from a drawable material, the fibre comprising:
a first channel (404, 604, 1404) extending between the inlet (4) and the outlet (6), the sensing chamber (8) being formed within the first channel (404, 604, 1404), the first channel (404, 604, 1404) comprising a microfluidic flow channel or groove; and
a second channel (406, 606) extending along the sensor (2), which second channel (406, 606) is operatively connected to the first channel (404, 604, 1404),
the sensing element (10) comprises an optical sensor comprising one or more sensing optical fibres (12) extending along the sensor (2) such that at least a part of the or each sensing optical fibre (12) is operatively connected to the first channel (404, 604, 1404) by being exposed within the sensing chamber (8),
the sensor (2) comprises a first pump operatively connected to the outlet (6) and configured to:
in a sensing mode, cause a fluid to flow through the sensor by entering the sensor via the inlet (4) and then pass through the first channel (404, 604, 1404), the second channel (406, 606) and the sensing chamber (8) in order that one or more variables of the fluid may be optically sensed and analysed by the one or more sensing optical fibres (12) of the sensing element (10); and
in a cleaning mode, flush a cleaning fluid through the first channel (404, 604, 1404) via the outlet (6) of the sensor (2).

2. A sensor (2) according to claim 1, further comprising a seal (610), sealingly connected to the first fibre (16).

3. A sensor (2) according to claim 1 or claim 2, wherein the first channel (404, 604, 1404) comprises patterns etched into the wall of the first channel (404, 604, 1404) in the sensing chamber (8).

4. A sensor (2) according to any one of the preceding claims, wherein the first pump comprises a syringe pump (414).

5. A sensor (2) according to any one of the preceding claims, wherein the first pump comprises a reservoir pump (2904), operatively connected to a reservoir (2900) holding fluid.

6. A sensor (2) according to any one of the preceding claims, comprising an electrical sensor (14) extending along the first fibre (16) such that at least a part of the electrical sensor (14) is operatively connected to the first channel (404, 604, 1404) by means of the sensing chamber (8).

7. A sensor (2) according to any one of the preceding claims, further comprising a second pump operatively connected to the second channel (406, 606).

8. A sensor (2) according to any one of the preceding claims, further comprising a third channel (1408), operatively connected to the first channel (404, 604, 1404) by means of the sensing chamber (8).

9. A sensor (2) according to any of the preceding claims, further wherein the sensing element (10) comprises a first probe element removably positioned within the first channel (404, 604, 1404).

10. A sensor (2) according to any one of the preceding claims, comprising a second drawn fibre adapted to be removably positionable within the second channel.

11. A sensor (2) according to claim 8 or any claim dependent thereon, wherein the sensor comprises a light sensitive material operatively connected to one or more of the first, second and third channels, which light sensitive material is optionally patterned with a micro-fluidic pattern and/or optionally forms an end portion of the sensor.

12. A sensor (2) according to any one of the preceding claims, comprising a switch adapted to switch the sensor between a sensing configuration and a cleaning configuration wherein the switch optionally comprising a first switching optical fibre and a second switching optical fibre, the first switching optical fibre being operatively connectable to a cleaning channel and the sensing element (10), and the second switching optical fibre being operatively connected to a drain channel and the sensing element (10).

13. A sensor (2) according to any one of the preceding claims, comprising a data analysis unit connected to a proximal end of the first fibre (16); wherein the one or more sensing optical fibres operatively connect the sensing element (10) to the data analysis unit.

## Patentansprüche

1. Sensor (2), umfassend einen Einlass (4) und einen Auslass (6), eine Erfassungskammer (8), die zwischen dem Einlass (4) und dem Auslass (6) angeordnet ist, und ein Erfassungselement (10), das funktionell mit der Erfassungskammer (8) verbunden ist, wobei:
der Sensor (2) eine erste Faser (16) umfasst, die aus einem verstreckbaren Material gebildet ist, die Faser umfassend:
einen ersten Kanal (404, 604, 1404), der sich zwischen dem Einlass (4) und dem Auslass (6) erstreckt, wobei die Erfassungskammer (8) innerhalb des ersten Kanals (404, 604, 1404) gebildet wird, wobei der erste Kanal (404, 604, 1404) einen mikrofluidischen Strömungskanal oder eine Rille umfasst; und
einen zweiten Kanal (406, 606), der sich entlang des Sensors (2) erstreckt, wobei der zweite Kanal (406, 606) operativ mit dem ersten Kanal (404, 604, 1404) verbunden ist, wobei das Erfassungselement (10) einen optischen Sensor umfasst, der eine oder mehrere optische Erfassungsfasern (12) umfasst, die sich entlang des Sensors (2) erstrecken, sodass mindestens ein Teil der oder jeder optischen Erfassungsfaser (12) operativ mit dem ersten Kanal (404, 604, 1404) verbunden ist, indem sie in der Erfassungskammer (8) freiliegen,
der Sensor (2) eine erste Pumpe umfasst, die operativ mit dem Auslass (6) verbunden und konfiguriert ist zum:
Veranlassen, dass in einem Erfassungsmodus ein Fluid durch den Sensor fließt, indem es über den Einlass (4) in den Sensor eintritt und dann durch den ersten Kanal (404, 604, 1404), den zweiten Kanal (406, 606) und die Erfassungskammer (8) strömt, damit eine oder mehrere Variablen des Fluids optisch erfasst und durch die eine oder mehrere optische Erfassungsfasern (12) des Erfassungselements (10) analysiert werden können; und
Spülen eines Reinigungsfluids in einem Reinigungsmodus durch den ersten Kanal (404, 604, 1404) über den Auslass (6) des Sensors (2).

2. Sensor (2) nach Anspruch 1, ferner umfassend eine Dichtung (610), die dichtend mit der ersten Faser (16) verbunden ist.

3. Sensor (2) nach Anspruch 1 oder 2, wobei der erste Kanal (404, 604, 1404) Muster umfasst, die in die Wand des ersten Kanals (404, 604, 1404) in der Erfassungskammer (8) geätzt sind.

4. Sensor (2) nach einem der vorstehenden Ansprüche, wobei die erste Pumpe eine Spritzenpumpe (414) umfasst.

5. Sensor (2) nach einem der vorstehenden Ansprüche, wobei die erste Pumpe eine Reservoirpumpe (2904) umfasst, die funktionell mit einem Reservoir (2900) verbunden ist, das Fluid enthält.

6. Sensor (2) nach einem der vorstehenden Ansprüche, umfassend einen elektrischen Sensor (14), der sich entlang der ersten Faser (16) erstreckt, sodass mindestens ein Teil des elektrischen Sensors (14) mittels der Erfassungskammer (8) operativ mit dem ersten Kanal (404, 604, 1404) verbunden ist.

7. Sensor (2) nach einem der vorstehenden Ansprüche, ferner umfassend eine zweite Pumpe, die betriebsmäßig mit dem zweiten Kanal (406, 606) verbunden ist.

8. Sensor (2) nach einem der vorstehenden Ansprüche, ferner umfassend einen dritten Kanal (1408), der über die Erfassungskammer (8) mit dem ersten Kanal (404, 604, 1404) in Wirkverbindung steht.

9. Sensor (2) nach einem der vorstehenden Ansprüche, wobei das Erfassungselement (10) ferner ein erstes Sondenelement umfasst, das abnehmbar in dem ersten Kanal (404, 604, 1404) angeordnet ist.

10. Sensor (2) nach einem der vorstehenden Ansprüche, umfassend eine zweite gezogene Faser, die in dem zweiten Kanal entfernbar positioniert werden kann.

11. Sensor (2) nach Anspruch 8 oder einem davon abhängigen Anspruch, wobei der Sensor ein lichtempfindliches Material umfasst, das operativ mit einem oder mehreren der ersten, zweiten und dritten Kanäle verbunden ist, wobei das lichtempfindliche Material optional mit einem mikrofluidischen Muster strukturiert ist und/oder optional einen Endabschnitt des Sensors bildet.

12. Sensor (2) nach einem der vorstehenden Ansprüche, umfassend einen Schalter, der geeignet ist, den Sensor zwischen einer Erfassungskonfiguration und einer Reinigungskonfiguration umzuschalten, wobei der Schalter optional eine erste schaltende optische Faser und eine zweite schaltende optische Faser umfasst, wobei die erste schaltende optische Faser operativ mit einem Reinigungskanal und dem Erfassungselement (10) verbunden werden kann, und die zweite schaltende optische Faser operativ mit einem Abflusskanal und dem Erfassungselement (10) verbunden werden kann.

13. Sensor (2) nach einem der vorstehenden Ansprüche, umfassend eine Datenanalyseeinheit, die mit einem proximalen Ende der ersten Faser (16) verbunden ist; wobei die eine oder mehreren optischen Erfassungsfasern das Erfassungselement (10) operativ mit der Datenanalyseeinheit verbinden.

## Revendications

1. Capteur (2) comprenant une entrée (4) et une sortie (6), une chambre de détection (8) positionnée entre l'entrée (4) et la sortie (6), et un élément de détection (10) relié fonctionnellement à la chambre de détection (8), dans lequel :
le capteur (2) comprend une première fibre (16) formée d'un matériau étirable, la fibre comprenant :
un premier canal (404, 604, 1404) s'étendant entre l'entrée (4) et la sortie (6), la chambre de détection (8) étant formée à l'intérieur du premier canal (404, 604, 1404), le premier canal (404, 604, 1404) comprenant un canal ou une rainure d'écoulement microfluidique ; et
un deuxième canal (406, 606) s'étendant le long du capteur (2), lequel deuxième canal (406, 606) est fonctionnellement relié au premier canal (404, 604, 1404),
l'élément de détection (10) comprend un capteur optique comprenant une ou plusieurs fibres optiques de détection (12) s'étendant le long du capteur (2) de sorte qu'au moins une partie de la ou de chaque fibre optique de détection (12) est fonctionnellement reliée au premier canal (404, 604, 1404) en étant exposée à l'intérieur de la chambre de détection (8),
le capteur (2) comprend une première pompe reliée fonctionnellement à la sortie (6) et configurée pour :
en mode de détection, amener un fluide à circuler à travers le capteur en entrant dans le capteur par l'intermédiaire de l'entrée (4) puis à passer à travers le premier canal (404, 604, 1404), le deuxième canal (406, 606) et la chambre de détection (8) afin qu'une ou plusieurs variables du fluide puissent être détectées et analysées optiquement par les une ou plusieurs fibres optiques de détection (12) de l'élément de détection (10) ; et
en mode nettoyage, faire passer un fluide de nettoyage à travers le premier canal (404, 604, 1404) par l'intermédiaire de la sortie (6) du capteur (2).

2. Capteur (2) selon la revendication 1, comprenant en outre un joint (610), relié de manière étanche à la première fibre (16).

3. Capteur (2) selon la revendication 1 ou la revendication 2, dans lequel le premier canal (404, 604, 1404) comprend des motifs gravés dans la paroi du premier canal (404, 604, 1404) dans la chambre de détection (8).

4. Capteur (2) selon l'une quelconque des revendications précédentes, dans lequel la première pompe comprend une pompe à seringue (414).

5. Capteur (2) selon l'une quelconque des revendications précédentes, dans lequel la première pompe comprend une pompe à réservoir (2904), reliée de manière fonctionnelle à un réservoir (2900) contenant du fluide.

6. Capteur (2) selon l'une quelconque des revendications précédentes, comprenant un capteur électrique (14) s'étendant le long de la première fibre (16) de sorte qu'au moins une partie du capteur électrique (14) est reliée de manière fonctionnelle au premier canal (404, 604, 1404) au moyen de la chambre de détection (8).

7. Capteur (2) selon l'une quelconque des revendications précédentes, comprenant en outre une seconde pompe reliée de manière fonctionnelle au deuxième canal (406, 606).

8. Capteur (2) selon l'une quelconque des revendications précédentes, comprenant en outre un troisième canal (1408), relié de manière fonctionnelle au premier canal (404, 604, 1404) au moyen de la chambre de détection (8).

9. Capteur (2) selon l'une quelconque des revendications précédentes, dans lequel, en outre, l'élément de détection (10) comprend un premier élément de sonde positionné de manière amovible à l'intérieur du premier canal (404, 604, 1404).

10. Capteur (2) selon l'une quelconque des revendications précédentes, comprenant une seconde fibre étirée adaptée pour être positionnable de manière amovible dans le deuxième canal.

11. Capteur (2) selon la revendication 8 ou toute revendication dépendante de celle-ci, dans lequel le capteur comprend un matériau sensible à la lumière relié de manière fonctionnelle à l'un ou à plusieurs des premier, deuxième et troisième canaux, lequel matériau sensible à la lumière est éventuellement modelé avec un motif microfluidique et/ou forme éventuellement une partie d'extrémité du capteur.

12. Capteur (2) selon l'une quelconque des revendications précédentes, comprenant un commutateur adapté pour commuter le capteur entre une configuration de détection et une configuration de nettoyage, dans lequel le commutateur comprend éventuellement une première fibre optique de commutation et une seconde fibre optique de commutation, la première fibre optique de commutation pouvant être reliée de manière fonctionnelle à un canal de nettoyage et à l'élément de détection (10), et la seconde fibre optique de commutation étant reliée de manière fonctionnelle à un canal de drainage et à l'élément de détection (10).

13. Capteur (2) selon l'une quelconque des revendications précédentes, comprenant une unité d'analyse de données reliée à une extrémité proximale de la première fibre (16) ; dans lequel les une ou plusieurs fibres optiques de détection relient de manière fonctionnelle l'élément de détection (10) à l'unité d'analyse de données.
